# FASCICULE DE BREVET EUROPEEN

(11) **EP 1 296 948 B1**
(45) Date de publication et mention de la délivrance du brevet: **10.09.2003**
(21) Numéro de dépôt: 01954060.8
(22) Date de dépôt: 06.07.2001
(51) Int. Cl.: C07D 209/42

(54) **NOUVELLE FORME CRISTALLINE GAMMA DU SEL DE TERT-BUTYLAMINE DU PERINDOPRIL, SON PROCEDE DE PREPARATION, ET LES COMPOSITIONS PHARMACEUTIQUES QUI LA CONTIENNENT**
NEUE KRISTALLINE FORM GAMMA DES TERT-BUTYLAMIN SALZES VON PERINDOPRIL, VERFAHREN ZU DEREN HERSTELLUNG UND DIESES ENTHALTENDE ARZNEIMITTEL
NOVEL GAMMA CRYSTALLINE FORM OF PERINDOPRIL TERT-BUTYLAMINE SALT, PREPARATION METHOD, AND PHARMACEUTICAL COMPOSITIONS CONTAINING SAME.

(30) Priorité: 06.07.2000 FR 0008791
(43) Date de publication de la demande: 02.04.2003
(73) Titulaire: Les Laboratoires Servier, 92415 Courbevoie Cedex (FR)
(72) Inventeur: PFEIFFER, Bruno, F-95320 Saint Leu la Forêt (FR); GINOT, Yves-Michel, F-45000 Orléans (FR); COQUEREL, Gérard, F-76520 Boos (FR); BEILLES, Stéphane, F-21000 DIJON (FR)
(86) Numéro de dépôt international: FR0102169
(87) Numéro de publication internationale: WO01083439

(56) Documents cités:
- EP-A- 0 308 341
- FR-A- 2 771 010

## Description

La présente invention concerne une nouvelle forme cristalline γ du sel de tert-butylamine du perindopril de formule (I): 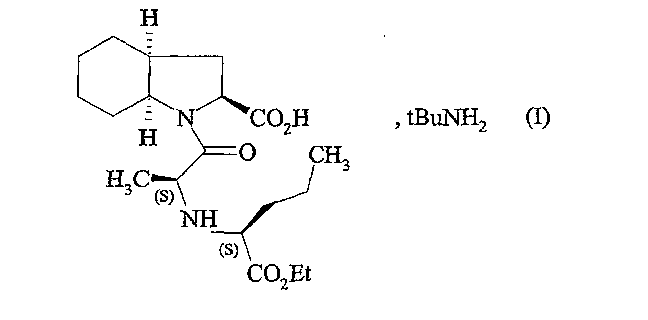 son procédé de préparation ainsi que les compositions pharmaceutiques qui la contiennent.

Le perindopril, ainsi que ses sels pharmaceutiquement acceptables, et plus particulièrement son sel de tert-butylamine, possèdent des propriétés pharmacologiques intéressantes.
Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte tenu de l'intérêt pharmaceutique de ce composé, il était primordial de l'obtenir avec une excellente pureté. Il était également important de pouvoir le synthétiser selon un procédé facilement transposable à l'échelle industrielle, et notamment sous une forme permettant une filtration et un séchage rapides. Enfin, cette forme devait être parfaitement reproductible, facilement formulée et suffisamment stable pour autoriser son stockage prolongé sans conditions particulières de température, de lumière, d'humidité ou de taux d'oxygène.

Le brevet EP 0 308 341 décrit un procédé de synthèse industrielle du perindopril. Cependant, ce document ne précise pas les conditions d'obtention du perindopril sous une forme présentant ces caractéristiques de manière reproductible.

La demanderesse a présentement trouvé qu'un sel particulier du perindopril, le sel de tert-butylamine, pouvait être obtenu sous une forme cristalline bien définie, parfaitement reproductible et présentant notamment des caractéristiques de formulation.

Plus spécifiquement, la présente invention concerne la forme cristalline γ du composé de formule (I), caractérisée par le diagramme de diffraction X sur poudre suivant, mesuré sur un diffractomètre Siemens D5005 (anticathode de cuivre) et exprimé en termes de distance inter-réticulaire d, d'angle de Bragg 2 thêta, d'intensité et d'intensité relative (exprimée en pourcentage par rapport à la raie la plus intense) :

| **Angle 2 thêta (°)** | **Distance inter- réticulaire d (Å)** | **Intensité** | **Intensité relative (%)** |
|---|---|---|---|
| 6,298 | 14,02 | 630 | 39,8 |
| 7,480 | 11,81 | 380 | 24 |
| 8,700 | 10,16 | 1584 | 100 |
| 9,276 | 9,53 | 318 | 20,1 |
| 10,564 | 8,37 | 526 | 33,2 |
| 11,801 | 7,49 | 54 | 3,4 |
| 12,699 | 6,96 | 86 | 5,4 |
| 13,661 | 6,48 | 178 | 11,2 |
| 14,095 | 6,28 | 163 | 10,3 |
| 14,332 | 6,17 | 290 | 18,3 |
| 14,961 | 5,92 | 161 | 10,2 |
| 15,793 | 5,61 | 128 | 8,1 |
| 16,212 | 5,46 | 179 | 11,3 |
| 16,945 | 5,23 | 80 | 5,1 |
| 17,291 | 5,12 | 92 | 5,8 |
| 17,825 | 4,97 | 420 | 26,5 |
| 18,100 | 4,90 | 159 | 10 |
| 18,715 | 4,74 | 89 | 5,6 |
| 19,017 | 4,66 | 118 | 7,4 |
| 19,362 | 4,58 | 134 | 8,5 |
| 19,837 | 4,47 | 133 | 8,4 |
| 20,609 | 4,31 | 95 | 6 |
| 21,232 | 4,18 | 257 | 16,2 |
| 21,499 | 4,13 | 229 | 14,5 |
| 21,840 | 4,07 | 127 | 8 |
| 22,129 | 4,01 | 191 | 12,1 |
| 22,639 | 3,92 | 137 | 8,6 |
| 23,000 | 3,86 | 88 | 5,6 |
| 23,798 | 3,74 | 147 | 9,3 |
| 24,170 | 3,68 | 70 | 4,4 |
| 25,066 | 3,55 | 167 | 10,5 |
| 25,394 | 3,50 | 165 | 10,4 |
| 26,034 | 3,42 | 84 | 5,3 |
| 26,586 | 3,35 | 75 | 4,7 |
| 27,541 | 3,24 | 74 | 4,7 |
| 28,330 | 3,15 | 85 | 5,4 |
| 29,589 | 3,02 | 96 | 6,1 |

L'invention s'étend également au procédé de préparation de la forme cristalline γ du composé de formule (I), caractérisé en ce que :
- soit, selon une première variante, on porte à reflux une solution du sel de tert-butylamine du perindopril dans le chloroforme, puis on refroidit rapidement la solution à 0°C et après agitation on collecte le solide obtenu par filtration,
- soit, selon une seconde variante, on porte à reflux une solution du sel de tert-butylamine du perindopril dans l'acétate d'éthyle, on refroidit rapidement la solution entre 0 et 5°C puis on collecte le solide ainsi obtenu par filtration. On met ce solide en suspension dans le chloroforme, on agite la suspension à température ambiante pendant 5 à 10 jours, puis on collecte le solide par filtration.
   - Dans le procédé de cristallisation selon l'invention, on peut utiliser le composé de formule (1) obtenu par n'importe quel procédé. Avantageusement, on utilise le composé de formule (I) obtenu par le procédé de préparation décrit dans le brevet EP 0 308 341.
   - Dans la première variante du procédé selon l'invention, la concentration du composé de formule (I) dans le chloroforme est préférentiellement comprise entre 150 et 300 g/l.
   - Dans la seconde variante du procédé selon l'invention, la concentration du composé de formule (I) dans l'acétate d'éthyle est préférentiellement comprise entre 70 et 90 g/l. La concentration du solide obtenu dans le chloroforme est préférentiellement comprise entre 100 et 150 g/l.

L'invention s'étend aussi aux compositions pharmaceutiques renfermant comme principe actif la forme cristalline γ du composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc.

La posologie utile est adaptable selon la nature et la sévérité de l'affection, la voie d'administration ainsi que l'âge et le poids du patient. Cette posologie varie de 1 à 500 mg par jour en une ou plusieurs prises.

Les compositions pharmaceutiques selon l'invention peuvent également contenir un diurétique comme l'indapamide.

Les exemples suivants illustrent l'invention mais ne la limitent en aucune façon.

Le spectre de diffraction X sur poudre a été mesuré avec les conditions expérimentales suivantes :
- Diffractomètre Siemens D5005, détecteur à scintillations,
- Anticathode de cuivre (λ=1,5405 Å), voltage 40 KV, intensité 40mA,
- Montage θ-θ,
- Domaine de mesures : 5° à 30°,
- Incrémentation entre chaque mesure : 0,02°,
- Temps de mesure par pas : 2s,
- Fentes variables : v6,
- Filtre Kβ (Ni),
- Pas de référence interne,
- Procédure de zéro avec les fentes Siemens,
- Données expérimentales traitées avec le logiciel EVA (version 5.0).

### EXEMPLE 1 : Forme cristalline γ du sel de tert-butylamine du perindopril

100 g du sel de tert-butylamine du perindopril obtenu selon le procédé décrit dans le brevet EP 0 308 341 sont dissous dans 500 ml de chloroforme portés au reflux.
La solution est ensuite refroidie à 0°C et agitée une nuit à cette température. Le solide obtenu est collecté par filtration.

### Diagramme de diffraction X sur poudre :

Le profil de diffraction des rayons X de la poudre (angles de diffraction) de la forme γ du sel de tert-butylamine du perindopril est donné par les raies significatives rassemblées dans le tableau suivant, avec l'intensité et l'intensité relative (exprimée en pourcentage par rapport à la raie la plus intense) :

| **Angle 2 thêta (°)** | **Distance inter- réticulaire d (Å)** | **Intensité** | **Intensité relative (%)** |
|---|---|---|---|
| 6,298 | 14,02 | 630 | 39,8 |
| 7,480 | 11,81 | 380 | 24 |
| 8,700 | 10,16 | 1584 | 100 |
| 9,276 | 9,53 | 318 | 20,1 |
| 10,564 | 8,37 | 526 | 33,2 |
| 11,801 | 7,49 | 54 | 3,4 |
| 12,699 | 6,96 | 86 | 5,4 |
| 13,661 | 6,48 | 178 | 11,2 |
| 14,095 | 6,28 | 163 | 10,3 |
| 14,332 | 6,17 | 290 | 18,3 |
| 14,961 | 5,92 | 161 | 10,2 |
| 15,793 | 5,61 | 128 | 8,1 |
| 16,212 | 5,46 | 179 | 11,3 |
| 16,945 | 5,23 | 80 | 5,1 |
| 17,291 | 5,12 | 92 | 5,8 |
| 17,825 | 4,97 | 420 | 26,5 |
| 18,100 | 4,90 | 159 | 10 |
| 18,715 | 4,74 | 89 | 5,6 |
| 19,017 | 4,66 | 118 | 7,4 |
| 19,362 | 4,58 | 134 | 8,5 |
| 19,837 | 4,47 | 133 | 8,4 |
| 20,609 | 4,31 | 95 | 6 |
| 21,232 | 4,18 | 257 | 16,2 |
| 21,499 | 4,13 | 229 | 14,5 |
| 21,840 | 4,07 | 127 | 8 |
| 22,129 | 4,01 | 191 | 12,1 |
| 22,639 | 3,92 | 137 | 8,6 |
| 23,000 | 3,86 | 88 | 5,6 |
| 23,798 | 3,74 | 147 | 9,3 |
| 24,170 | 3,68 | 70 | 4,4 |
| 25,066 | 3,55 | 167 | 10,5 |
| 25,394 | 3,50 | 165 | 10,4 |
| 26,034 | 3,42 | 84 | 5,3 |
| 26,586 | 3,35 | 75 | 4,7 |
| 27,541 | 3,24 | 74 | 4,7 |
| 28,330 | 3,15 | 85 | 5,4 |
| 29,589 | 3,02 | 96 | 6,1 |

### EXEMPLE 2 : Forme cristalline γ du sel de tert-butylamine du perindopril

125 g du sel de tert-butylamine du perindopril obtenu selon le procédé décrit dans le brevet EP 0 308 341 sont dissous dans 1,5 1 d'acétate d'éthyle portés au reflux.
La température de la solution est ensuite ramenée rapidement entre 0 et 5 °C.
Le solide obtenu est ensuite collecté par filtration, puis il est mis en suspension dans 750 g de chloroforme. La suspension est agitée à température ambiante pendant 5 à 10 jours puis le solide est collecté par filtration.

### EXEMPLE 3 : Composition pharmaceutique

| Formule de préparation pour 1000 comprimés dosés à 4 mg : | |
|---|---|
| Composé de l'exemple 1 | 4 g |
| Hydroxypropylcellulose | 2 g |
| Amidon de blé | 10 g |
| Lactose | 100 g |
| Stéarate de magnésium | 3 g |
| Talc | 3 g |

## Revendications

1. Forme cristalline γ du composé de formule (I) : 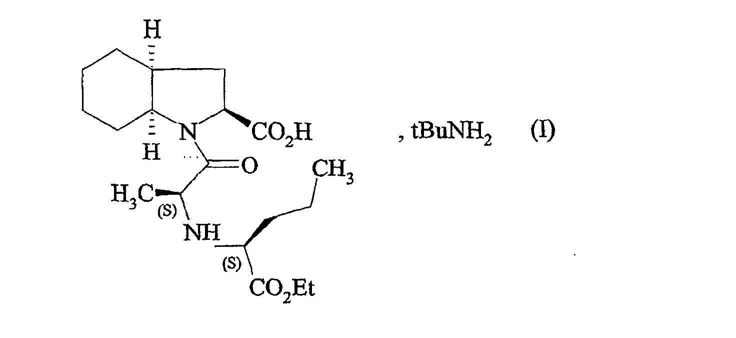 **caractérisée par** le diagramme de diffraction X sur poudre suivant, mesuré sur un diffractomètre (anticathode de cuivre) et exprimé en termes de distance inter-réticulaire d, d'angle de Bragg 2 thêta, d'intensité et d'intensité relative (exprimée en pourcentage par rapport à la raie la plus intense) :
| **Angle 2 thêta (°)** | **Distance inter- réticulaire d (Å)** | **Intensité** | **Intensité relative (%)** |
|---|---|---|---|
| 6,298 | 14,02 | 630 | 39,8 |
| 7,480 | 11,81 | 380 | 24 |
| 8,700 | 10,16 | 1584 | 100 |
| 9,276 | 9,53 | 318 | 20,1 |
| 10,564 | 8,37 | 526 | 33,2 |
| 11,801 | 7,49 | 54 | 3,4 |
| 12,699 | 6,96 | 86 | 5,4 |
| 13,661 | 6,48 | 178 | 11,2 |
| 14,095 | 6,28 | 163 | 10,3 |
| 14,332 | 6,17 | 290 | 18,3 |
| 14,961 | 5,92 | 161 | 10,2 |
| 15,793 | 5,61 | 128 | 8,1 |
| 16,212 | 5,46 | 179 | 11,3 |
| 16,945 | 5,23 | 80 | 5,1 |
| 17,291 | 5,12 | 92 | 5,8 |
| 17,825 | 4,97 | 420 | 26,5 |
| 18,100 | 4,90 | 159 | 10 |
| 18,715 | 4,74 | 89 | 5,6 |
| 19,017 | 4,66 | 118 | 7,4 |
| 19,362 | 4,58 | 134 | 8,5 |
| 19,837 | 4,47 | 133 | 8,4 |
| 20,609 | 4,31 | 95 | 6 |
| 21,232 | 4,18 | 257 | 16,2 |
| 21,499 | 4,13 | 229 | 14,5 |
| 21,840 | 4,07 | 127 | 8 |
| 22,129 | 4,01 | 191 | 12,1 |
| 22,639 | 3,92 | 137 | 8,6 |
| 23,000 | 3,86 | 88 | 5,6 |
| 23,798 | 3,74 | 147 | 9,3 |
| 24,170 | 3,68 | 70 | 4,4 |
| 25,066 | 3,55 | 167 | 10,5 |
| 25,394 | 3,50 | 165 | 10,4 |
| 26,034 | 3,42 | 84 | 5,3 |
| 26,586 | 3,35 | 75 | 4,7 |
| 27,541 | 3,24 | 74 | 4,7 |
| 28,330 | 3,15 | 85 | 5,4 |
| 29,589 | 3,02 | 96 | 6,1 |

2. Procédé de préparation de la forme cristalline γ du composé de formule (I) selon la revendication 1, **caractérisé en ce que** l'on porte à reflux une solution du sel de tert-butylamine du perindopril dans le chloroforme, puis on refroidit la solution à 0°C et collecte le solide obtenu par filtration.

3. Procédé de préparation de la forme cristalline γ du composé de formule (I) selon la revendication 1, **caractérisé en ce que** l'on porte à reflux une solution du sel de tert-butylamine du perindopril dans l'acétate d'éthyle, on refroidit rapidement la solution puis on collecte le solide ainsi obtenu par filtration, on le met en suspension dans le chloroforme, on agite la suspension à température ambiante pendant 5 à 10 jours, puis on collecte le solide par filtration.

4. Procédé selon l'une quelconque des revendications 2 ou 3, **caractérisé en ce que** l'on utilise le composé de formule (I) obtenu par le procédé de préparation décrit dans le brevet EP 0 308 341.

5. Procédé selon la revendication 2, **caractérisé en ce que** la concentration du composé de formule (I) dans le chloroforme est comprise entre 150 et 300 g/l.

6. Procédé selon la revendication 3, **caractérisé en ce que** la concentration du composé de formule (I) dans l'acétate d'éthyle est comprise entre 70 et 90 g/l.

7. Composition pharmaceutique contenant comme principe actif le composé selon la revendication 1, en combinaison avec un ou plusieurs véhicules inertes, non toxiques et pharmaceutiquement acceptables.

8. Composition pharmaceutique selon la revendication 7 utile pour la fabrication de médicaments utiles en tant qu'inhibiteur de l'enzyme de conversion de l'angiotensine I.

9. Composition pharmaceutique selon la revendication 8 utile pour la fabrication de médicaments utiles dans le traitement des maladies cardiovasculaires.

10. Composition pharmaceutique selon l'une quelconque des revendications 7 à 9 **caractérisée en ce qu'**elle contient également un diurétique.

11. Composition pharmaceutique selon la revendication 10 **caractérisée en ce que** le diurétique est l'indapamide.

## Claims

1. γ crystalline form of the compound of formula (I) : 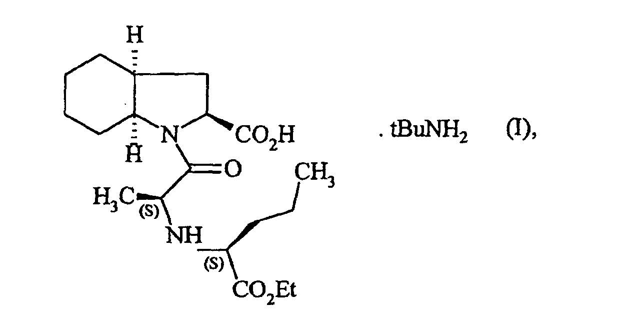 **characterised by** the following powder X-ray diffraction diagram, measured using a diffractometer (copper anticathode) and expressed in terms of inter-planar distance d, Bragg's angle 2 theta, intensity and relative intensity (expressed as a percentage with respect to the most intense ray) :
| **Angle 2 theta (°)** | **Inter-planar distance d (Å)** | **Intensity** | **Relative intensity (%)** |
|---|---|---|---|
| 6.298 | 14.02 | 630 | 39.8 |
| 7.480 | 11.81 | 380 | 24 |
| 8.700 | 10.16 | 1584 | 100 |
| 9.276 | 9.53 | 318 | 20.1 |
| 10.564 | 8.37 | 526 | 33.2 |
| 11.801 | 7.49 | 54 | 3.4 |
| 12.699 | 6.96 | 86 | 5.4 |
| 13.661 | 6.48 | 178 | 11.2 |
| 14.095 | 6.28 | 163 | 10.3 |
| 14.332 | 6.17 | 290 | 18.3 |
| 14.961 | 5.92 | 161 | 10.2 |
| 15.793 | 5.61 | 128 | 8.1 |
| 16.212 | 5.46 | 179 | 11.3 |
| 16.945 | 5.23 | 80 | 5.1 |
| 17.291 | 5.12 | 92 | 5.8 |
| 17.825 | 4.97 | 420 | 26.5 |
| 18.100 | 4.90 | 159 | 10 |
| 18.715 | 4.74 | 89 | 5.6 |
| 19.017 | 4.66 | 118 | 7.4 |
| 19.362 | 4.58 | 134 | 8.5 |
| 19.837 | 4.47 | 133 | 8.4 |
| 20.609 | 4.31 | 95 | 6 |
| 21.232 | 4.18 | 257 | 16.2 |
| 21.499 | 4.13 | 229 | 14.5 |
| 21.840 | 4.07 | 127 | 8 |
| 22.129 | 4.01 | 191 | 12.1 |
| 22.639 | 3.92 | 137 | 8.6 |
| 23.000 | 3.86 | 88 | 5.6 |
| 23.798 | 3.74 | 147 | 9.3 |
| 24.170 | 3.68 | 70 | 4.4 |
| 25.066 | 3.55 | 167 | 10.5 |
| 25.394 | 3.50 | 165 | 10.4 |
| 26.034 | 3.42 | 84 | 5.3 |
| 26.586 | 3.35 | 75 | 4.7 |
| 27.541 | 3.24 | 74 | 4.7 |
| 28.330 | 3.15 | 85 | 5.4 |
| 29.589 | - 3.02 | 96 | 6.1 |

2. Process for the preparation of the γ crystalline form of the compound of formula (I) according to claim 1, **characterised in that** a solution of perindopril tert-butylamine salt in chloroform is heated at reflux, the solution is then cooled to 0°C and the solid obtained is collected by filtration.

3. Process for the preparation of the γ crystalline form of the compound of formula (I) according to claim 1, **characterised in that** a solution of perindopril tert-butylamine salt in ethyl acetate is heated at reflux, the solution is rapidly cooled, the solid thereby obtained is then collected by filtration, it is suspended in chloroform, the suspension is stirred at ambient temperature for from 5 to 10 days, and the solid is then collected by filtration.

4. Process according to either claim 2 or claim 3, **characterised in that** the compound of formula (I) obtained by the preparation process described in patent specification EP 0 308 341 is used.

5. Process according to claim 2, **characterised in that** the concentration of the compound of formula (I) in the chloroform is from 150 to 300 g/litre.

6. Process according to claim 3, **characterised in that** the concentration of the compound of formula (I) in the ethyl acetate is from 70 to 90 g/litre.

7. Pharmaceutical composition comprising as active ingredient the compound according to claim 1, in combination with one or more pharmaceutically acceptable, inert, nontoxic carriers.

8. Pharmaceutical composition according to claim 7 for use in the manufacture of medicaments for use as inhibitors of angiotensin I converting enzyme.

9. Pharmaceutical composition according to claim 8 for use in the manufacture of medicaments for use in the treatment of cardiovascular diseases.

10. Pharmaceutical composition according to any one of claims 7 to 9, **characterised in that** it also comprises a diuretic.

11. Pharmaceutical composition according to claim 10, **characterised in that** the diuretic is indapamide.

## Patentansprüche

1. γ-Kristallform der Verbindung der Formel (I): 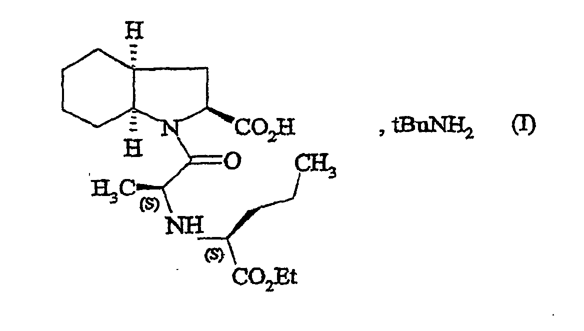 **gekennzeichnet durch** das nachfolgende Pulver-Röntgenbeugungsdiagramm, welches mit Hilfe eines Diffraktometers (Kupfer-Antikathode) gemessen worden und **durch** die Gitterabstände, die Bragg-Winkel 2 Theta, die Intensität und die relative Intensität (ausgedrückt als Prozentsatz bezogen auf die Bande mit der höchsten Intensität) angegeben ist:
| **2 Theta-Winkel (°)** | **Gitterabstand d (Å)** | **Intensität** | **Relative Inten- sität (%)** |
|---|---|---|---|
| 6,298 | 14,02 | 630 | 39,8 |
| 7,480 | 11,81 | 380 | 24 |
| 8,700 | 10,16 | 1584 | 100 |
| 9,276 | 9,53 | 318 | 20,1 |
| 10,564 | 8,37 | 526 | 33,2 |
| 11,801 | 7,49 | 54 | 3,4 |
| 12,699 | 6,96 | 86 | 5,4 |
| 13,661 | 6,48 | 178 | 11,2 |
| 14,095 | 6,28 | 163 | 10,3 |
| 14,332 | 6,17 | 290 | 18,3 |
| 14,961 | 5,92 | 161 | 10,2 |
| 15,793 | 5,61 | 128 | 8,1 |
| 16,212 | 5,46 | 179 | 11,3 |
| 16,945 | 5,23 | 80 | 5,1 |
| 17,291 | 5,12 | 92 | 5,8 |
| 17,825 | 4,97 | 420 | 26,5 |
| 18,100 | 4,90 | 159 | 10 |
| 18,715 | 4,74 | 89 | 5,6 |
| 19,017 | 4,66 | 118 | 7,4 |
| 19,362 | 4,58 | 134 | 8,5 |
| 19,837 | 4,47 | 133 | 8,4 |
| 20,609 | 4,31 | 95 | 6 |
| 21,232 | 4,18 | 257 | 16,2 |
| 21,499 | 4,13 | 229 | 14,5 |
| 21,840 | 4,07 | 127 | 8 |
| 22,129 | 4,01 | 191 | 12,1 |
| 22,639 | 3,92 | 137 | 8,6 |
| 23,000 | 3,86 | 88 | 5,6 |
| 23,798 | 3,74 | 147 | 9,3 |
| 24,170 | 3,68 | 70 | 4,4 |
| 25,066 | 3,55 | 167 | 10,5 |
| 25,394 | 3,50 | 165 | 10,4 |
| 26,034 | 3,42 | 84 | 5,3 |
| 26,586 | 3,35 | 75 | 4,7 |
| 27,541 | 3,24 | 74 | 4,7 |
| 28,330 | 3,15 | 85 | 5,4 |
| 29,589 | 3,02 | 96 | 6,1 |

2. Verfahren zur Herstellung der γ-Kristallform der Verbindung der Formel (I) nach Anspruch 1, **dadurch gekennzeichnet, daß** man eine Lösung des tert.-Butylaminsalzes von Perindopril in Chloroform zum Sieden am Rückfluß erhitzt, die Lösung dann auf 0°C abkühlt und den erhaltenen Feststoff durch Filtration gewinnt.

3. Verfahren zur Herstellung der γ-Kristallform der Verbindung der Formel (I) nach Anspruch 1, **dadurch gekennzeichnet, daß** man eine Lösung des tert.-Butylaminsalzes von Perindopril in Ethylacetat zum Sieden am Rückfluß erhitzt, die Lösung schnell abkühlt und den in dieser Weise erhaltenen Feststoff durch Filtration gewinnt, ihn in Chloroform suspendiert, die Suspension während 5 bis 10 Tagen bei Raumtemperatur rührt und dann den Feststoff durch Filtration gewinnt.

4. Verfahren nach einem der Ansprüche 2 oder 3, **dadurch gekennzeichnet, daß** man die Verbindung der Formel (I) verwendet, die man nach dem Herstellungsverfahren erhalten hat, das in dem Europäischen Patent EP 0 308 341 beschrieben ist.

5. Verfahren nach Anspruch 2, **dadurch gekennzeichnet, daß** die Konzentration der Verbindung der Formel (I) in Chloroform zwischen 150 und 300 g/l liegt.

6. Verfahren nach Anspruch 3, **dadurch gekennzeichnet, daß** die Konzentration der Verbindung der Formel (I) In Ethylacetat zwischen 70 und 90 g/l liegt.

7. Pharmazeutische Zubereitung enthaltend als Wirkstoff die Verbindung nach Anspruch 1 in Kombination mit einem oder mehreren inerten, nichttoxischen und pharmazeutisch annehmbaren Trägermaterialien.

8. Pharmazeutische Zubereitung nach Anspruch 7 für die Herstellung von Arzneimitteln, die als Inhibitoren des Angiotensin I umwandelnden Enzyms geeignet sind.

9. Pharmazeutische Zubereitung nach Anspruch 8 für die Herstellung von Arzneimitteln, die für die Behandlung von kardiovaskulären Erkrankungen geeignet sind.

10. Pharmazeutische Zubereitung nach einem der Ansprüche 7 bis 9, **dadurch gekennzeichnet, daß** sie zusätzlich ein Diuretikum enthält.

11. Pharmazeutische Zubereitung nach Anspruch 10, **dadurch gekennzeichnet, daß** das Diuretikum Indapamid ist.
